# EUROPEAN PATENT SPECIFICATION

(11) **EP 3 316 947 B1**
(45) Date of publication and mention of the grant of the patent: **04.09.2019**
(21) Application number: 16728655.8
(22) Date of filing: 02.06.2016
(51) Int. Cl.: A61M 15/00

(54) **DRY POWDER INHALER**
TROCKENPULVERINHALATOR
INHALATEUR À POUDRE SÈCHE

(30) Priority: 03.07.2015 SE 1550964
(43) Date of publication of application: 09.05.2018
(73) Proprietor: Iconovo AB, 223 63 Lund (SE)
(72) Inventor: LASTOW, Orest, 247 45 Torna Hällestad (SE); ARVIDSSON, Lars, 247 51 Dalby (SE)
(74) Representative: Ström & Gulliksson AB
(86) International application number: PCT/EP2016/062512
(87) International publication number: WO 2017/005420

(56) References cited:
- WO-A1-2014/204417
- US-A1- 2005 279 357
- US-A1- 2013 125 885
- US-A1- 2013 255 679
- US-A1- 2014 318 538

## Description

### Field of the Invention

This invention pertains in general to the field of medicament inhalers, and more particularly to capsule-based dry powder inhalers.

### Background of the Invention

Inhalers have been widely used in the pharmaceutical field for treatment of respiratory and/or other diseases. Numerous drugs, medications and other substances are inhaled into the lungs using the inhalers for rapid absorption of the drug etc. in the blood stream and for local action in the lung.

Inhaled drugs fall into two main categories, one being in the form of liquids, including suspensions, and the other being powders. The choice of liquids or powders depends on the characteristics of the drugs, medications, etc. to be inhaled.

The most common type of inhaler is the pressurized metered-dose inhaler. In this type of inhaler medication is most commonly stored in solution in a pressurized canister that contains a propellant, although it may also be a suspension. The canister is attached to a plastic, hand-operated actuator. On activation, the metered-dose inhaler releases a fixed dose of medication in aerosol form.

Another kind of inhaler is a nebulizer, which supplies medication as an aerosol created from an aqueous formulation.

The kind referred to herein is yet another type in the form of a dry powder inhaler and in particular, a capsule-based dry powder inhaler. A dry powder inhaler releases a measured dose of powdered medication that is inhaled through the inhaler. In a capsule-based dry powder inhaler the medication is stored in a capsule inside the inhaler. The capsule is pierced to release the medication prior to inhalation by the user.

US 5,947,118 discloses a capsule holder for use with an inhaler. The capsule holder has at least three ribs for clamping the pharmaceutical capsules so that the capsules do not fall out during normal handling of the inhaler. The inhaler has a lid that is connected to a lower part via a hinge. The lid is flipped shut to close off and prevent dust from entering the inhaler.

US 7,252,087 B2 relates to an inhaler for inhaling powdered medicament from capsules inserted in a capsule holder before use. The inhaler has a lid and a lower part connected via a hinge. In use the lid and lower part are separated from each other by a force introduced through a multi-functioning actuating member rather than a force applied directly to the lid or lower part.

The inhalers disclosed in US 5,947,118 and US 7,252,087 B2 have complex designs with numerous parts thereby leading to difficulties in their manufacture. For instance, the hinge of the US 7,252,087 B2 inhaler requires a separate hinge bolt and an elastic spring to assist pivoting of the mouthpiece. There remains a need for a dry powder inhaler having a simpler design while maintaining optimal performance during use.

Other inhalers with separate covers are, for example, disclosed in EP1270034 and EP1747036.

US 2013/255679 A1 discloses a dry powder inhaler with the opposite hinge arrangement to that claimed. US 2005/279357 A1 discloses a dry powder inhaler with a hinge bolt to which a cover, mouthpiece and plate are all pivotally attached. US 2013/125885 A1 discloses a dry powder inhaler which uses individual blisters. The mouth piece pivots partially to allow insertion of the blister and is then returned to position for inhalation.

### Summary of the Invention

Accordingly, the present invention preferably seeks to mitigate, alleviate or eliminate one or more of the above-identified deficiencies in the art and disadvantages singly or in any combination and solves at least the above mentioned problems by providing in one aspect a dry powder inhaler comprising: a cover comprising at least one cover hinge member; a mouthpiece comprising a shaft receivable by the cover hinge member; and a container for the dry powder, the container comprising a container hinge member for receiving the shaft; wherein the cover hinge member, shaft, and container hinge member form a hinge about which the cover and the mouthpiece are pivotable.

Further advantageous embodiments are disclosed in the appended and dependent patent claims.

### Brief Description of the Drawings

These and other aspects, features and advantages of which the invention is capable of will be apparent and elucidated from the following description of embodiments of the present invention with reference to the accompanying drawings, wherein:
Fig. 1 shows separated and perspective views of a cover, mouthpiece, and container of the inhaler according to one embodiment of the present invention;
Fig. 2 is a perspective view showing a cover about to be connected to one end of the mouthpiece in Fig. 1, wherein a cross-sectional perspective view of the cover is shown for clarity, according to one embodiment of the present invention;
Fig. 3 is a perspective view showing the cover connected to one end of the mouthpiece in Fig. 1, wherein a cross-sectional perspective view of the cover is again shown for clarity, according to one embodiment of the present invention;
Fig. 4 is a perspective view of the cover connected to one end of a mouthpiece shown in Fig. 3 with the container about to be connected to the same end, wherein a cross-sectional perspective view of the cover is shown for clarity, according to one embodiment of the present invention;
Fig. 5 is the perspective view of Fig. 4 showing a full cover, according to one embodiment of the present invention;
Fig. 6 is a perspective view showing a cover and a container connected to the mouthpiece in Fig. 1 to form an assembled inhaler, according to one embodiment, wherein a cross-sectional perspective view of the cover is shown for clarity;
Fig. 7 is a perspective view of an assembled inhaler in Fig. 6, showing the full cover, according to one embodiment of the present invention;
Fig. 8 is a perspective view of an inhaler in a closed position according to one embodiment of the present invention;
Fig. 9 is a perspective view of the inhaler in Fig. 8 with a cover open to expose a mouthpiece, according to one embodiment of the present invention;
Fig. 10 is a perspective view of the inhaler in Fig. 9 with a cover open and a mouthpiece unfastened from a container, according to one embodiment of the present invention; and
Fig. 11 is a perspective view of an inhaler in an open position to show the interior of the inhaler's container according to one embodiment of the present invention.

### Description of embodiments

The following description focuses on an embodiment of the present invention applicable to a medicament inhaler, and in particular to a dry powder inhaler suitable for use with capsule-based medicaments. However, it will be appreciated that the invention is not limited to these applications but may be applied to many other inhalers having a cover, a mouthpiece, and a medicament container.

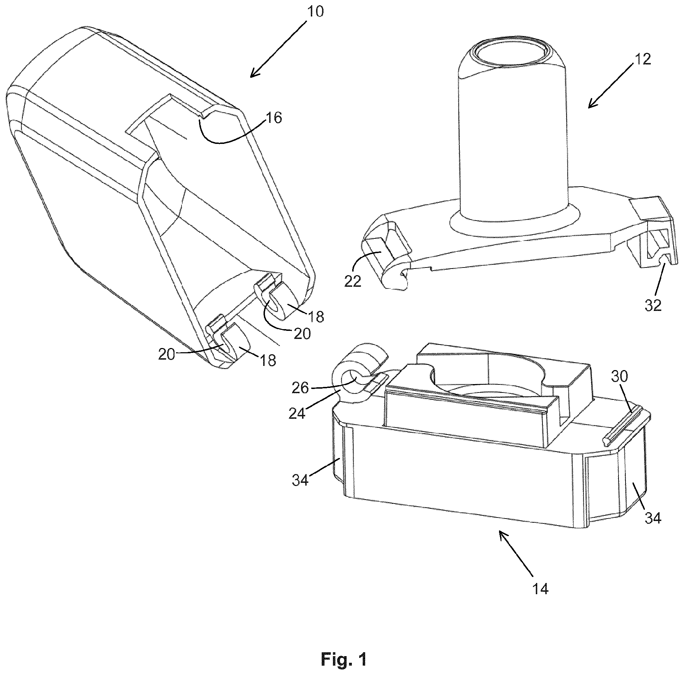

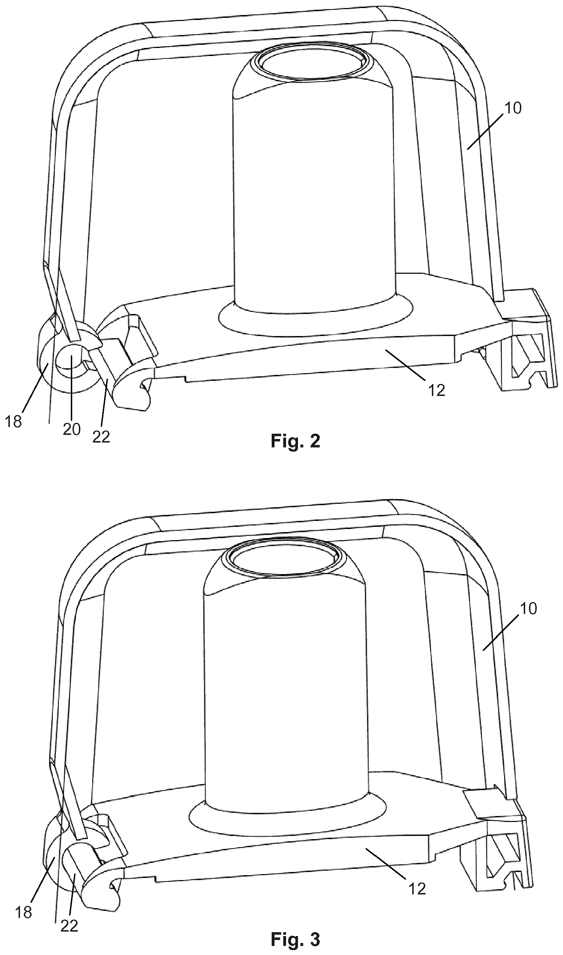

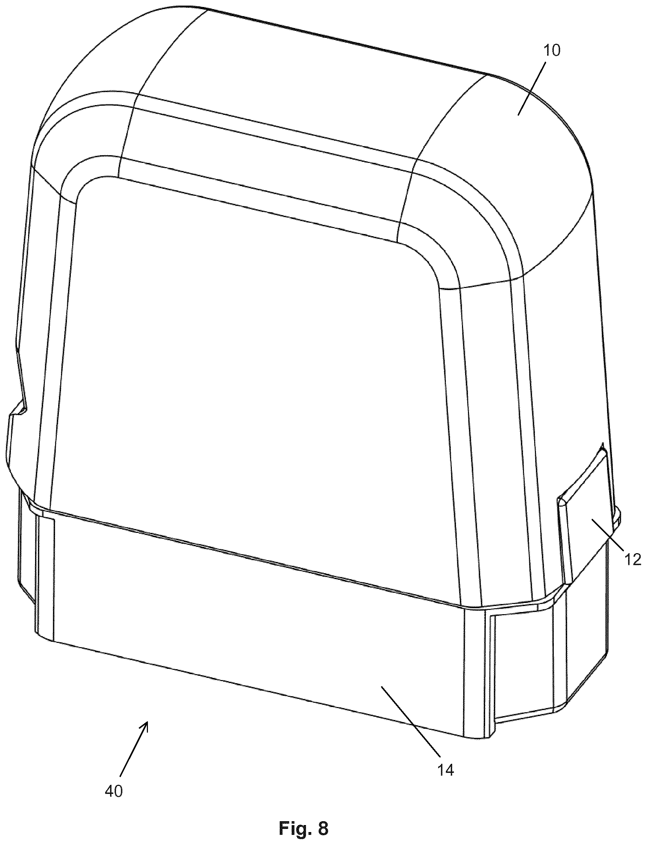

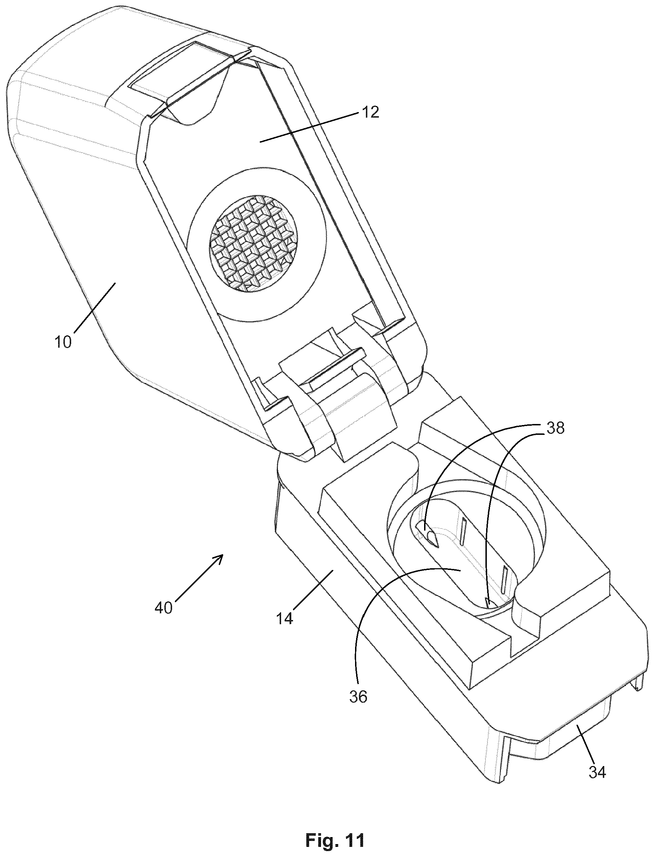

Figs. 1 to 11 illustrate a dry powder inhaler 40 according to one embodiment of the invention. Fig. 1 illustrates the three major components of the dry powder inhaler 40. Figs. 2 to 7 illustrate the assembly of the inhaler 40 from the three major components. Figs. 8 to 10 demonstrate how the assembled inhaler 40 is handled. Fig. 11 illustrates the interior of the container for the medicament.

Referring to Fig. 1, the dry powder inhaler comprises a cover 10, mouthpiece 12, and a container 14 all of which may be manufactured from any suitable material for example, injection moldable plastics such as thermoplastics.

Cover 10 comprises cover hinge members 18 each with a cover hinge recess 20 for receiving a beveled shaft on the mouthpiece 12, as will be described with respect to Figs. 2 to 7. The cover hinge members 18 are located at one end of the cover 10 and equidistant from the cover's longitudinal plane of symmetry. Although two cover hinge members 18 are illustrated in Fig. 1, it will be understood that only one cover hinge member 18 may be present on the cover 10. Alternatively, three or more cover hinge members 18 may be included. Similarly, the cover hinge member or members 18 could be located on, or immediately adjacent to, the cover's longitudinal plane of symmetry. The number and arrangement of cover hinge members 18 will depend on the number and arrangement of container hinge members on the container 14 as described below. On an end opposing the hinge members 18, cover 10 comprises a cover fastening recess 16 for reversibly receiving a fastening member on the container 14. Cover 10 encloses the mouthpiece 12 to prevent impurities such as dust from coming into contact with the mouthpiece 12 and/or entering the container 14 when the inhaler is not in use as will become evident from the description below.

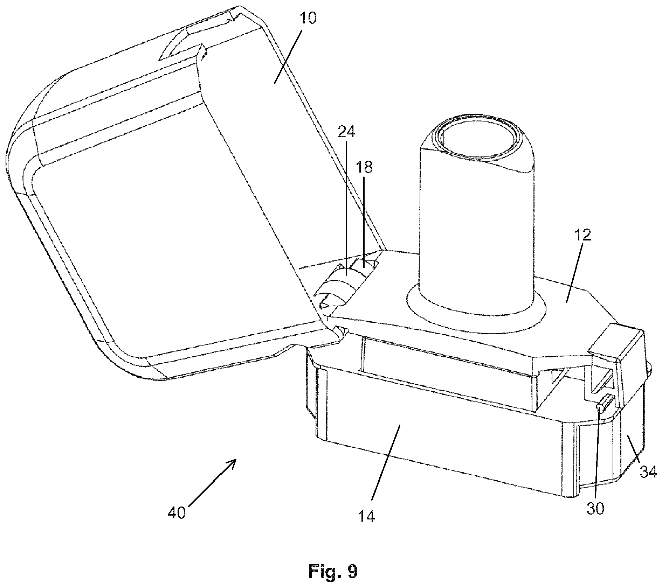

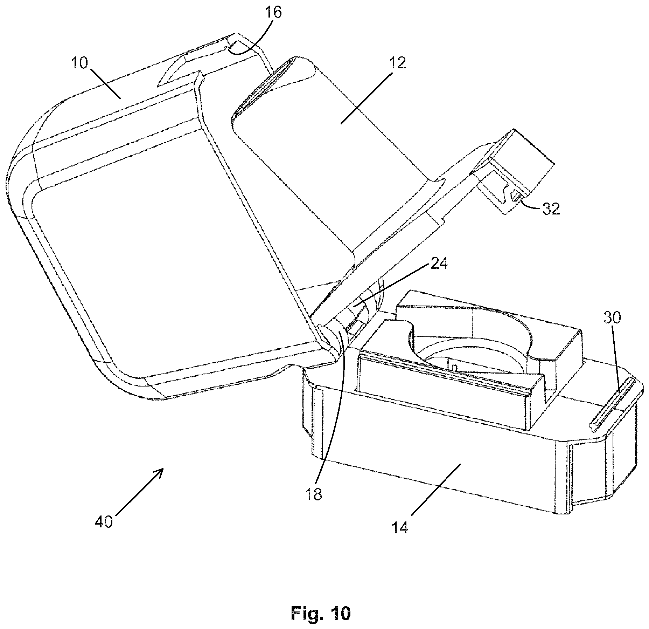

Mouthpiece 12 comprises a beveled shaft 22 at one end of the mouthpiece 12. The shaft may be beveled at any suitable angle. For instance, the shaft could be chamfered i.e. at an angle of 45 degrees. More than one beveled surface may be present on the shaft 22 and each surface may be beveled at differing angles. In the assembled inhaler, beveled shaft 22 is received by both the cover hinge recess 20 and a container hinge recess to form a hinge at one end of the inhaler. The beveled shaft 22 facilitates uncomplicated assembly of the inhaler and prevents cover 10 and mouthpiece 12 from becoming separated from the inhaler when they are pivoted about the hinge as described below with respect to Figs. 9 and 10. Shaft 22 could be manufactured separately then attached to the mouthpiece 12 but is preferably integrally molded as part of the mouthpiece 12 during its manufacture. Mouthpiece 12 also comprises a mouthpiece fastening recess 32 on an end opposing beveled shaft 22 for reversibly receiving a fastening member on the container 14.

The medicament, for example the dry powder and/or the pharmaceutical capsule(s), is located in the interior of the container 14 prior to inhalation of the medicament as will be described further below in Fig. 11. At one end, container 14 comprises container hinge member 24 with a container hinge recess 26 for receiving the beveled shaft 22 of mouthpiece 12. More than one container hinge member 24 may be present. This will depend on the number and arrangement of the cover hinge members 18 on the cover 10 as described above. If two container hinge members 24 are present they may be symmetrically located to each other about a plane of symmetry on the container. On an opposing end, container 14 comprises fastening member 30 that is reversibly receivable by both cover fastening recess 16 and mouthpiece fastening recess 32. Although member 30 resembles an elongated ridge, the member 30 could instead be made up of several knobs having complementary recesses on the cover and mouthpiece. Container 14 further comprises at least one capsule piercing actuator 34 for piercing a capsule inside the container 14 to release a powder for inhalation (as described further below with reference to Fig. 11).

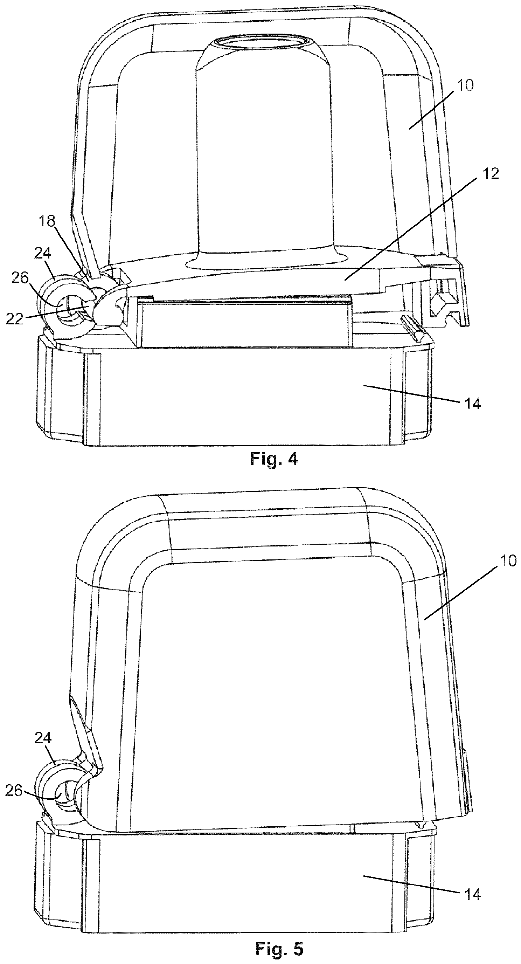

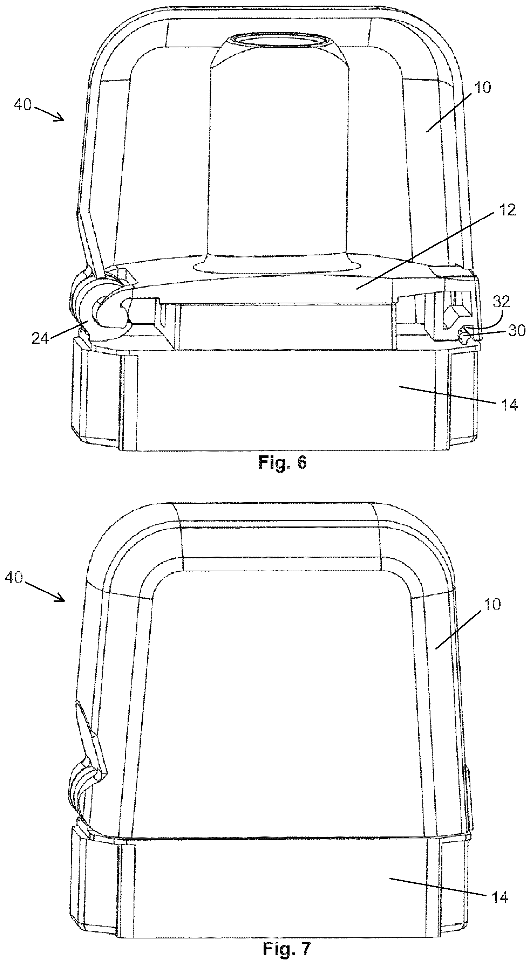

The inhaler 40 is assembled as follows. Cover 10 is placed over mouthpiece 12 in order that cover hinge member 18 is brought into close proximity with beveled shaft 22 (Fig. 2). Beveled shaft 22 is then inserted into cover hinge recess 20 to connect the cover to the mouthpiece (Fig. 3). Container 14 is then placed underneath the connected cover and mouthpiece with the container hinge member 24 in close proximity to the beveled shaft 22 (Figs. 4 and 5). The beveled shaft 22 is then inserted into container hinge recess 26 and the fastening member 30 is snapped into both cover fastening recess 16 and mouthpiece fastening recess 32 to form the dry powder inhaler 40 (Figs. 6 and 7). It is possible to disassemble the inhaler 40 by performing the steps in Figs. 2 to 7 in the reverse order. Disassembling the inhaler may be desirable for cleaning, refilling, or disposal purposes.

When the inhaler 40 is not in use for example, during storage or transport, it remains in the closed position illustrated in Fig. 8. In the closed position the cover 10, mouthpiece 12, and container 14 are securely fastened together at one end of the inhaler by the engagement of the fastening member 30 with the cover fastening recess 16 and mouthpiece fastening recess 32. This prevents the cover and the mouthpiece from slipping out of the hinge formed by cover hinge members 18, container hinge member 24, and beveled shaft 22 at the opposing end of the inhaler.

In all positions other than the closed position (for example, the open positions in Figs. 9 and 10), the beveled shaft 22 is locked in, and thus cannot be removed from, both the cover hinge member 18 and the container hinge member 24. This locking of the hinge prevents detachment of the cover 10 and mouthpiece 12 from the inhaler as the cover 10 and mouthpiece 12 pivot about the hinge.

To use the inhaler 40 one applies a force with their thumb or fingers at one end of the cover 10 to detach the cover from the fastening member 30 on the container 14. The mouthpiece 12 remains fastened to the container 14 as shown in Fig. 9. The cover is then pivoted away from the mouthpiece about the hinge at the other end of the inhaler. If the inhaler contains a capsule the user then depresses capsule piercing actuator 34 to pierce the capsule and release the powdered medicament. The user brings the mouthpiece to their mouth and inhales the powder. The cover 10 may then be pivoted back over mouthpiece 12 and re-fastened to fastening member 30 thereby closing the inhaler for storage and later use.

The inhaler 40 may be cleaned and/or re-filled with powder or capsules by first detaching the cover 10 at one end as described above with respect to Fig. 9 then applying force to the mouthpiece 12 at the same end to unfasten the mouthpiece from the fastening member 30. Like the cover 10, the mouthpiece 12 is then pivoted about the hinge at the other end of the inhaler thereby exposing the interior of the container 14 as illustrated in Fig. 10. After cleaning and/or refilling the inhaler, the mouthpiece is pivoted back and re-fastened to fastening member 30 on container 14. The inhaler is optionally used before cover 10 is pivoted back over the mouthpiece 12 and attached to fastening member 30.

The interior of the container 14 is illustrated in Fig. 11. The interior comprises capsule bay 36 and needles 38 for piercing the capsule (not shown). Needles 38 are located on opposing sides of the capsule bay 36 and operate by a spring-loaded mechanism in that the needles move into capsule bay 36 to pierce the capsule when capsule piercing actuator(s) 34 are depressed. Upon release of actuator(s) 34, the needles 38 withdraw from capsule bay 36 into the housing of the container 14.

Although, the present invention has been described above with reference to specific embodiments, it is not intended to be limited to the specific form set forth herein. Rather, the invention is limited only by the accompanying claims.

In the claims, the term "comprises/comprising" does not exclude the presence of other elements or steps. Furthermore, although individually listed, a plurality of means, elements or method steps may be implemented by e.g. a single unit or processor. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc do not preclude a plurality. Reference signs in the claims are provided merely as a clarifying example and shall not be construed as limiting the scope of the claims in any way.

## Claims

1. A dry powder inhaler (40) comprising:
a cover (10) comprising at least one cover hinge member (18);
a mouthpiece (12) comprising a shaft (22) receivable by the cover hinge member (18); and
a container (14) for the dry powder, the container (14) comprising a container hinge member (24) for receiving the shaft (22);
wherein the cover hinge member (18), shaft (22), and container hinge member (24) form a hinge about which the cover (10) and the mouthpiece (12) are pivotable.

2. The inhaler according to claim 1, wherein the shaft (22) is beveled.

3. The inhaler according to claim 1 or 2, wherein the cover (10) further comprises a fastening recess (16), the container (14) comprises a fastening member (30) and the mouthpiece (12) comprises a mouthpiece fastening recess (32), wherein the fastening member (30) is received by the cover fastening recess (16) and the mouthpiece fastening recess (32) when the inhaler (40) is in a closed position, and
wherein the cover fastening recess (16) is detached from the fastening member (30) when the inhaler (40) is in a first open position, and the mouthpiece fastening recess (32) is detached from the fastening member (30) when the inhaler (40) is in a second open position.

4. The dry powder inhaler (40) according to any one of claims 1 to 3, comprising two cover hinge members (18) located at one end of the cover (10) and equidistant from a plane of symmetry of the cover (10).

5. The dry powder inhaler (40) according to any claim 3, wherein the cover fastening recess (16) and cover hinge member(s) (18) are located on opposing ends of the cover (10).

6. The dry powder inhaler (40) according to any claim 2, wherein the shaft (22) has more than one beveled surface, preferably two beveled surfaces.

7. The dry powder inhaler (40) according to any one of claims 1 to 6, wherein the shaft (22) is chamfered.

8. The dry powder inhaler (40) according to claim 3, wherein the mouthpiece fastening recess (32) and shaft (22) are located on opposing ends of the mouthpiece (12).

9. The dry powder inhaler (40) according to claim 3, wherein the fastening member (30) and container hinge member (24) are located on opposing ends of the container (14).

10. The dry powder inhaler (40) according to claim 3, wherein the fastening member (30) is an elongated ridge.

11. The dry powder inhaler (40) according to any one of claims 1 to 10, wherein the container (14) further comprises at least one capsule piercing actuator (34).

12. The dry powder inhaler (40) according to any one of claims 1 to 11, wherein the container (14) further comprises two capsule piercing actuators (34), each of the actuators (34) being located at opposing ends of the container (14).

13. The dry powder inhaler (40) according to any one of claims 1 to 12, wherein the container (14) further comprises at least one needle (38) for piercing a capsule in the interior of the container.

14. The dry powder inhaler (40) according to any one of claims 1 to 13, wherein the cover (10), mouthpiece (12), and container (14) are manufactured from injection moldable plastics.

15. The dry powder inhaler (40) according to any of claims 1 to 14, wherein the dry powder inhaler (40) is a capsule-based dry powder inhaler.

## Patentansprüche

1. Ein Trockenpulverinhalator (40) mit:
einer Abdeckung (10) mit zumindest einem Abdeckungsscharnierelement (18),
einem Mundstück (12) mit einem durch das Abdeckungsscharnierelement (18) aufnehmbaren Schaft (22), und
einem Behälter (14) für das Trockenpulver, wobei der Behälter (14) ein Behälter-Scharnierelement (24) zur Aufnahme des Schafts (22) aufweist,
wobei das Abdeckungs-Scharnierelement (18), der Schaft (22) und das Behälter-Scharnierelement (24) ein Scharnier bilden, um das die Abdeckung (10) und das Mundstück (12) schwenkbar sind.

2. Der Inhalator gemäß Anspruch 1, wobei der Schaft (22) kegelförmig bzw. abgeschrägt ist.

3. Der Inhalator gemäß Anspruch 1 oder 2, wobei die Abdeckung (10) ferner eine Befestigungsausnehmung (16) aufweist, der Behälter (14) ein Befestigungselement (30) aufweist, und das Mundstück (12) eine Mundstück-Befestigungsausnehmung (32) aufweist, wobei das Befestigungselement (30) durch die Abdeckungs-Befestigungsausnehmung (16) und die Mundstück-Befestigungsausnehmung (32) aufgenommen ist, wenn der Inhalator (40) in einer geschlossenen Position ist, und
wobei die Abdeckungs-Befestigungsausnehmung (16) von dem Befestigungselement (30) abgenommen ist, wenn der Inhalator (40) in einer ersten offenen Position ist, und die Mundstück-Befestigungsausnehmung (32) von dem Befestigungselement (30) abgenommen ist, wenn der Inhalator (40) in einer zweiten offenen Position ist.

4. Der Trockenpulverinhalator (40) gemäß einem der Ansprüche 1 bis 3, mit zwei Abdeckungs-Scharnierelementen (18), die sich an einem Ende der Abdeckung (10) und äquidistant von einer Symmetrieebene der Abdeckung (10) befinden.

5. Der Trockenpulverinhalator (40) gemäß einem Anspruch 3, wobei die Abdeckungs-Befestigungsausnehmung (16) und das/die Abdeckungs-Scharnierelement/Scharnierelemente (18) sich an gegenüberliegenden Enden der Abdeckung (10) befinden.

6. Der Trockenpulverinhalator (40) gemäß einem Anspruch 2, wobei der Schaft (22) mehr als eine kegelförmige bzw. abgeschrägte Oberfläche, vorzugsweise zwei kegelförmige bzw. abgeschrägte Oberflächen besitzt.

7. Der Trockenpulverinhalator (40) gemäß einem der Ansprüche 1 bis 6, wobei der Schaft (22) angefast ist.

8. Der Trockenpulverinhalator (40) gemäß Anspruch 3, wobei die Mundstück-Befestigungsausnehmung (32) und der Schaft (22) sich an gegenüberliegenden Enden des Mundstücks (12) befinden.

9. Der Trockenpulverinhalator (40) gemäß Anspruch 3, wobei das Befestigungselement (30) und das Behälter-Scharnierelement (24) sich an gegenüberliegenden Enden des Behälters (14) befinden.

10. Der Trockenpulverinhalator (40) gemäß Anspruch 3, wobei das Befestigungselement (30) ein länglicher Rand oder eine längliche Kante ist.

11. Der Trockenpulverinhalator (40) gemäß einem der Ansprüche 1 bis 10, wobei der Behälter (14) zumindest einen Kapsel-Einstechbetätiger (34) aufweist.

12. Der Trockenpulverinhalator (40) gemäß einem der Ansprüche 1 bis 11, wobei der Behälter (14) ferner zwei Kapsel-Einstechbetätiger (34) aufweist, wobei sich jeder der Betätiger (34) an gegenüberliegenden Enden des Behälters (14) befindet.

13. Der Trockenpulverinhalator (40) gemäß einem der Ansprüche 1 bis 12, wobei der Behälter (14) ferner zumindest eine Nadel (38) zum Einstechen einer Kapsel im Inneren des Behälters aufweist.

14. Der Trockenpulverinhalator (40) gemäß einem der Ansprüche 1 bis 13, wobei die Abdeckung (10), das Mundstück (12) und der Behälter (14) aus spritzgießformbarem Kunststoff hergestellt sind.

15. Der Trockenpulverinhalator (40) gemäß einem der Ansprüche 1 bis 14, wobei der Trockenpulverinhalator (40) ein kapselbasierter Trockenpulverinhalator ist.

## Revendications

1. Inhalateur à poudre sèche (40) comprenant :
un couvercle (10) comprenant au moins un organe charnière de couvercle (18) ;
un embout buccal (12) comprenant une tige (22) pouvant être reçue par l'organe charnière de couvercle (18) ; et
un contenant (14) pour la poudre sèche, le contenant (14) comprenant un organe charnière de contenant (24) destiné à recevoir la tige (22) ;
dans lequel l'organe charnière de couvercle (18), la tige (22), et l'organe charnière de contenant (24) forment une charnière autour de laquelle le couvercle (10) et l'embout buccal (12) peuvent pivoter.

2. Inhalateur selon la revendication 1, dans lequel la tige (22) est biseautée.

3. Inhalateur selon la revendication 1 ou 2, dans lequel le couvercle (10) comprend en outre un évidement d'attache (16), le contenant (14) comprend un organe d'attache (30) et l'embout buccal (12) comprend un évidement d'attache d'embout buccal (32), dans lequel l'organe d'attache (30) est reçu par l'évidement d'attache de couvercle (16) et l'évidement d'attache d'embout buccal (32) lorsque l'inhalateur (40) est dans une position fermée, et
dans lequel l'évidement d'attache de couvercle (16) est détaché de l'organe d'attache (30) lorsque l'inhalateur (40) est dans une première position ouverte, et l'évidement d'attache d'embout buccal (32) est détaché de l'organe d'attache (30) lorsque l'inhalateur (40) est dans une seconde position ouverte.

4. Inhalateur à poudre sèche (40) selon l'une quelconque des revendications 1 à 3, comprenant deux organes charnières de couvercle (18) situés au niveau d'une extrémité du couvercle (10) et équidistants par rapport à un plan de symétrie du couvercle (10).

5. Inhalateur à poudre sèche (40) selon une quelconque revendication 3, dans lequel l'évidement d'attache de couvercle (16) et un ou des organes charnières de couvercle (18) sont situés sur des extrémités opposées du couvercle (10).

6. Inhalateur à poudre sèche (40) selon une quelconque revendication 2, dans lequel la tige (22) comporte plus d'une surface biseautée, de préférence deux surfaces biseautées.

7. Inhalateur à poudre sèche (40) selon l'une quelconque des revendications 1 à 6, dans lequel la tige (22) est chanfreinée.

8. Inhalateur à poudre sèche (40) selon la revendication 3, dans lequel l'évidement d'attache d'embout buccal (32) et la tige (22) sont situés sur des extrémités opposées de l'embout buccal (12).

9. Inhalateur à poudre sèche (40) selon la revendication 3, dans lequel l'organe d'attache (30) et l'organe charnière de contenant (24) sont situés sur des extrémités opposées du contenant (14).

10. Inhalateur à poudre sèche (40) selon la revendication 3, dans lequel l'organe d'attache (30) est une arête allongée.

11. Inhalateur à poudre sèche (40) selon l'une quelconque des revendications 1 à 10, dans lequel le contenant (14) comprend en outre au moins un actionneur de perçage de capsule (34).

12. Inhalateur à poudre sèche (40) selon l'une quelconque des revendications 1 à 11, dans lequel le contenant (14) comprend en outre deux actionneurs de perçage de capsule (34), chacun des actionneurs (34) étant situé au niveau d'extrémités opposées du contenant (14).

13. Inhalateur à poudre sèche (40) selon l'une quelconque des revendications 1 à 12, dans lequel le contenant (14) comprend en outre au moins une aiguille (38) destinée à percer une capsule dans l'intérieur du contenant.

14. Inhalateur à poudre sèche (40) selon l'une quelconque des revendications 1 à 13, dans lequel le couvercle (10), l'embout buccal (12), et le contenant (14) sont fabriqués à partir de plastiques moulables par injection.

15. Inhalateur à poudre sèche (40) selon l'une quelconque des revendications 1 à 14, dans lequel l'inhalateur à poudre sèche (40) est un inhalateur à poudre sèche à base de capsule.
